# EUROPEAN PATENT APPLICATION

(11) **EP 4 258 278 A1**
(43) Date of publication of application: **11.10.2023**
(21) Application number: 23165483.1
(22) Date of filing: 30.03.2023
(51) Int. Cl.: G16H 20/70, G16H 50/30, G16H 50/80, A47G 1/00, G09B 19/00

(54) **HYGIENE SYSTEM**

(30) Priority: 08.04.2022 US 202263328924 P; 24.03.2023 US 202318125922
(71) Applicant: Kohler Co., Kohler, WI 53044 (US)
(72) Inventor: MIXON, Delia, Suwanee, 30024 (US); ALLIS, Shane, Kohler, 53044 (US); BANCKS, Kathryn, Milwaukee, 53210 (US); ESTIBAN, Marwan, Mequon, 53097 (US); LEICHTY, Ryan, Sheboygan, 53083 (US); KWACZ, Jason, Kohler, 53044 (US); FAGG, Alexander, Burton-on-the-Water, GL54 2AN (GB); ERICKSON, Perry, Sheboygan, 53083 (US); DIEMEL, Douglas, Kohler, 53044 (US); KAPAL, Kenneth, Kohler, 53044 (US); TARPLEE, Jennifer, Sheboygan Falls, 53085 (US); SMITH, Joel, Cedarburg, 53012 (US); LAMPEN, Lowell, Mequon, 53092 (US); FRYE, Jacob, Sheboygan, 53085 (US); RUAN, Ellynna, Kohler, 53044 (US)
(74) Representative: Barker Brettell LLP

(57) **Abstract**

An apparatus for monitoring hygiene includes at least a communication interface configured to receive at least one hygiene criterion from a mobile device associated with a supervising user and receive hygiene performance data related to a hygiene activity a participating user via a sensor and a controller configured to perform an analysis the hygiene performance data according to at least one hygiene criterion and calculate a score for the participating user based on the analysis of the hygiene performance data.

## Description

### CROSS-REFERENCE TO RELATED APPLICATIONS

This application claims priority benefit of Provisional Application No. 63/328,924 filed April 8, 2022, and US Patent Application No. 18/125,922 filed March 24, 2023, which are hereby incorporated by reference in their entirety.

### FIELD

The present application relates generally to the improvement of hygiene in a bathroom or similar setting.

### BACKGROUND

Recent events have focused new attention on viral infections and the efficacy of hand washing and other hygiene practices for the prevention of spreading infection. Infections are a persistent problem in the healthcare industry that costs thousands of lives and billions of dollars annually. Improper hygiene is one of the biggest factors contributing to the transfer of germs and bacteria. The World Health Organization has issued guidelines to ensure proper hand hygiene that include recommend hand washing practices to mitigate the spread of infection. However, challenged remain in improvement of compliance and effectiveness in existing hygiene systems.

### BRIEF DESCRIPTION OF THE DRAWINGS

Exemplary embodiments are described herein with reference to the following drawings, according to an exemplary embodiment.
FIG. 1 illustrates an example hygiene reporting system.
FIG. 2 illustrates example computing devices for the hygiene reporting system.
FIG. 3 illustrates another example hygiene reporting system.
FIG. 4 illustrates another example hygiene reporting system.
FIG. 5 illustrates another example hygiene reporting system.
FIG. 6 illustrates example computing devices for the hygiene reporting system.
FIG. 7 illustrates an example mirror for the hygiene reporting system.
FIG. 8 illustrates another example mirror for the hygiene reporting system.
FIG. 9 illustrates another example mirror for the hygiene reporting system.
FIG. 10 illustrates another example mirror for the hygiene reporting system.
FIG. 11 illustrates an example sequence of actions for hygiene.
FIG. 12 illustrates another example hygiene reporting system.
FIG. 13 illustrates an example interface for a hygiene reporting system.
FIG. 14 illustrates an example supervising interface for a hygiene reporting system.
FIG. 15 illustrates another example supervising interface for a hygiene reporting system.
FIG. 16 illustrates an example participating interface for a hygiene reporting system.
FIG. 17 illustrates another example participating interface for a hygiene reporting system.
FIG. 18 illustrates a controller.
FIG. 19 illustrates a flow chart for the hygiene reporting system.
FIG. 20 illustrates an example automated handwashing system.
FIG. 21 illustrates a flow chart for the automated handwashing system.
FIG. 22 illustrates an example air current system.
FIG. 23 illustrates another view of the air current system.
FIG. 24 illustrates a flow chart for the air curtain system.
FIG. 25 illustrates an example view of the air current system to isolate specific users.

### DETAILED DESCRIPTION

The following embodiments include systems for reporting hygienic practices in a bathroom or similar space. In some examples, the user is instructed to follow certain procedures or motivated to follow certain procedures through direct instructions, awards, or a scoring technique. In other examples, apparatus are described to facilitate certain hygienic practices (e.g., handwashing). In other examples, systems are described to control air flows for sanitation purposes.

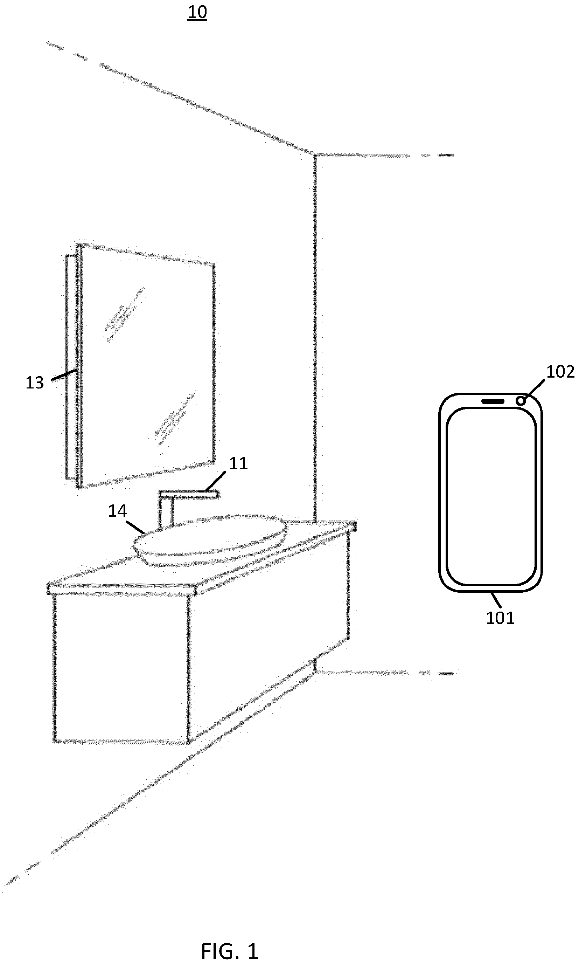

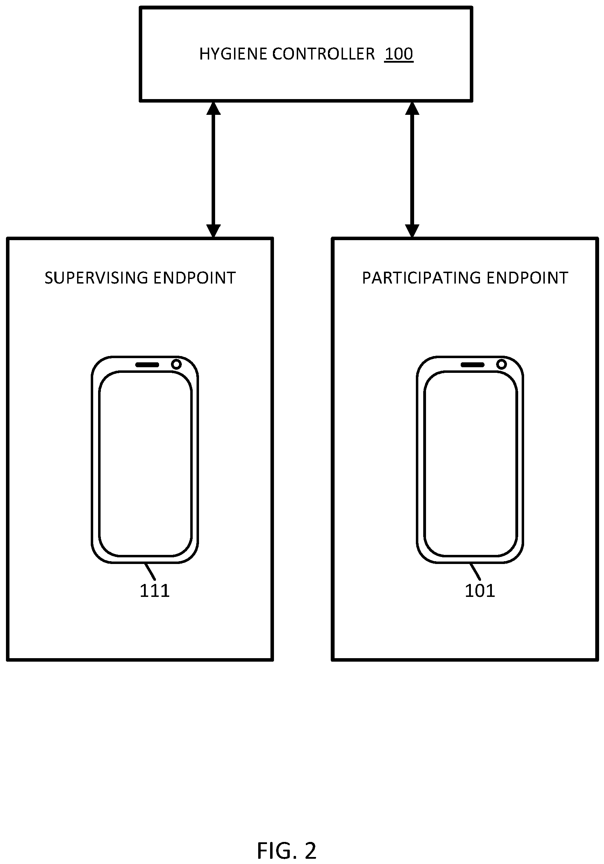

FIG. 1 illustrates an example hygiene reporting system in a bathroom or lavatory setting 10 for various embodiments of hygiene tracking and reporting. FIG. 2 illustrates example computing devices for the hygiene reporting system. The bathroom setting 10 may be included in a patient's room in a hospital or other health care facility. The bathroom setting 10 may be included in a surgical area (e.g., a lavatory or scrub sink adjacent to an operating room). The bathroom setting 10 may be included in a home or business.

The system may include a first mobile device as a participating endpoint 101 and a second mobile device as a supervising endpoint 111. The supervising endpoint 111 and the participating endpoint 101 may send and receive data with a hygiene controller 100. The hygiene controller 100 is in communication with at least one device in the bathroom setting 10. The bathroom setting 10 may include a data collection fixture such as a sink including a faucet 11 and a basin 14, and/or a mirror 13. At some times, the bathroom setting 10 may also include a mobile device (mobile data collection). The hygiene controller 100 may collect data from any of these devices and determine whether one or more hygiene requirements are being met by a user.

At least one device in the bathroom setting 10 may include a flow sensor. Data from the flow sensor allows the hygiene controller 100 to monitor practices in the bathroom setting 10. In some examples, the faucet 11 or plumbing leading to the faucet 11 includes a flow sensor that measures the flow of water to the faucet 11. In some examples, the plumbing leading away from the drain includes a flow sensor that measures the flow of water away from the basin. In other examples, flow of another liquid such as soap or a cleaning agent may be measured. Additional, different, or fewer components may be included.

The flow sensor may be a pressure sensor, ultrasonic sensor, or light sensor. The light sensor may measure the quantity of water that passes a light beam. The pressure sensor may detect the pressure of water inside a hose, a pipe or other plumbing device. The flow sensor may include two or more pressure sensors located at different places along the faucet 11 or plumping leading to the faucet 11. For example, the flow sensor may include a downstream pressure sensor and an upstream pressure sensor. The hygiene controller 100 may calculate the flow between the downstream pressure sensor and the upstream pressure sensor based on pressure measurements made by the respective sensors. The change in pressure may be proportional to the flow rate. The calculated flow may also be proportional to the size of the pipe (e.g., the square of the radius). The calculated flow rate may also depend on viscosity.

The ultrasonic sensor generates an ultrasonic wave that travels through the flow of water and is received at a received. Based on the received ultrasonic wave the volume and/or speed of the flow of water is detected. The sensor may be paired with two polished surface that reflects the ultrasonic wave or the light beam on the opposite side of the flow of water and returns the ultrasonic wave or the light beam to the sensor.

As an alternative to the flow sensor, the flow of water and/or soap may be determined according to the operation of a valve. For example, a solenoid may open and close the flow of water or soap. The hand hygiene controller 100 may control the solenoid to actuate in order to open or close the valve, for example, using an open command (e.g., energize the solenoid) and a close command (e.g., deenergize the solenoid). The open command, or turning on the faucet 11, may be generated in response to a user command. The user command may be a mechanical button (e.g., move the faucet handle or knob) or a motion sensor (e.g., wave an arm or hand in front of the motion sensor). The hand hygiene controller 100 may calculate a flow rate for the faucet 11 using a time difference between the open command and the closed command. The hygiene controller 100 may include a lookup table that relates the amount of time that the faucet 11 is on to flow amounts. In some examples, the time between the open and close commands is a predetermined time period, and in turn, the flow rate for one operation of the faucet 11 is a set amount.

The hygiene controller 100 may receive the flow sensor data and determine hygiene compliance. The hygiene controller 100 may determine whether a hygiene event has met one or more hygiene thresholds. The hygiene threshold may be a time of water flow for the faucet 11. The handwashing threshold may be a time duration or volumetric total of soap dispensed for a soap dispenser. The handwashing threshold may be a sequence for the faucet 11 and the soap dispenser. An example sequence may be water for a particular duration or amount then soap for a particular duration. An example sequence may be soap then water. An example sequence may be water for a particular duration or amount then soap then water for a particular duration. Additional water/soap usage sequences are described below.

Returning to FIG. 2, the first mobile device as a participating endpoint 101 and the second mobile device as a supervising endpoint 111 may send and receive data with the hygiene controller 100. In one example scenario, the participating endpoint 101 is assigned to a child and the supervising endpoint 111 is assigned to a parent or guardian. The hygiene reporting system allows certain inputs to be provided to the supervising endpoint 111 to define hygiene requirements for the child. In some instances, the hygiene practices of the child are monitored or measured by the participating endpoint 101, and in other instances, the hygiene practices of the child are monitored or measured by the participant endpoint 101 by other data collection devices. In either instance, the participating endpoint 101 and/or the supervising endpoint 111 may track the progress of the hygiene requirements and report the status of the hygiene requirements to the respective user.

The supervising endpoint 111 may receive inputs from the supervising user to define at least one hygiene criterion. The supervising endpoint 111 may send data representing the inputs to the hygiene controller 100. Example hygiene criteria may include whether or not a particular device has been used, the duration that a particular device was used, or a sequence of multiple devices. The at least one hygiene criterion may include a time value for the hygiene activity of the participating user. The time value may be a duration or a time of day (e.g., schedule). The hygiene criteria may measure handwashing, toothbrushing, flossing, face washing, or other hygiene activities.

The devices that may be required by the hygiene criteria include operation of the faucet 11, flushing a toilet, operation of an electric toothbrush, or other devices. The hygiene criteria may be a binary test identifying whether or not the device was operated by the participating user. The hygiene criteria may also define a time window for the operation of the device. For example, the hygiene criteria may define a window between a start window time (e.g., 7:30 pm) and an end window time (e.g., 8:00 pm) for the faucet 11. In order to satisfy this hygiene criteria, the participating user must operate the designated device during the time window.

The hygiene criteria may include a duration of the specified device. For example, the hygiene criteria may require that the device remain in operation for at least a predetermined time or no more than a predetermined time. The hygiene criteria may define a handwashing duration such as 10 seconds or 20 seconds for operation of the faucet 11. The hygiene criteria may define a toothbrushing duration such as 2 minutes for operation of the electric toothbrush. When the hygiene criteria specifies a maximum time, the purpose may be conservation of water. When the hygiene criteria specifies a minimum time, the purpose may be efficacy of the hygiene activity.

The hygiene criteria may include a predetermined sequence for multiple devices. The predetermine sequence may define that first device was operated before a second device. Each device in the sequence may be associated with a time window and/or a duration. An example sequence may be the electric toothbrush operated for at least 1 minute followed by the operation of the faucet 11 for at least 1 second.

The hygiene controller 100 may relay the hygiene criteria to the participating endpoint 101 and analysis may occur at the participating endpoint 101. The hygiene controller 100 receive hygiene performance data collected at the participating endpoint and analysis may occur at the hygiene controller 100. The hygiene performance data is related to hygiene activity by the participating and detected via a sensor. As described above, the sensor may include one or more cameras, a radar device, one or more flow sensors, and/or one or more proximity sensors. The hygiene performance data, as collected by a camera, may indicate the movement of the user or a particular lever, actuator, or button. The hygiene performance data indicate that the user is washing hands, brushing teeth, or performing another action. The hygiene performance data, as collected by a flow sensor, indicates that water is in use at a particular device. The hygiene performance data, as collected by the proximity sensor, may indicate the presence of the user.

The analysis of the hygiene performance data may be performed in according to at least one hygiene criterion. For example, the hygiene controller 100 may compare the hygiene criteria to the hygiene performance data. When the hygiene criteria includes whether or not a particular device has been used, the comparison may compare the usage data from a flow sensor for a particular device in the hygiene performance data to a specific device listed in the hygiene criteria. When the hygiene criteria includes duration that a particular device was used, the comparison may compare one or more timestamps from the hygiene performance data to the duration listed in the hygiene criteria. When the hygiene criteria includes a sequence of multiple devices, the comparison may compare the list of devices in the hygiene criteria to the usage data in the hygiene performance data.

The hygiene controller 100 may compare the hygiene criteria to the hygiene performance data in order to calculate a score for the participating user. The score may be a numerical representation of the extent that the hygiene criteria were met by the hygiene performance data.

For example, a numerical value may be assigned to each of the hygiene criteria. When there are five criteria, each may be assigned a value of one. Thus, when three criteria are met, the calculated score is 3 out of 5. In other examples, weights may be used such that a first criterion is assigned a first weight, a second criterion is assigned a second weight, and so on.

In other examples, a first weight is assigned to usage of a particular device, and a second weight is assigned to a duration. Thus, the score is a first value when the particular device is first used and increases over time as the device is under further use.

The hygiene controller 100 may receive at least one weight from the supervising endpoint 111 including a designation of one or more criteria and/or one or more time durations to be assigned each weight. Additional examples for the score and weighted components are described below.

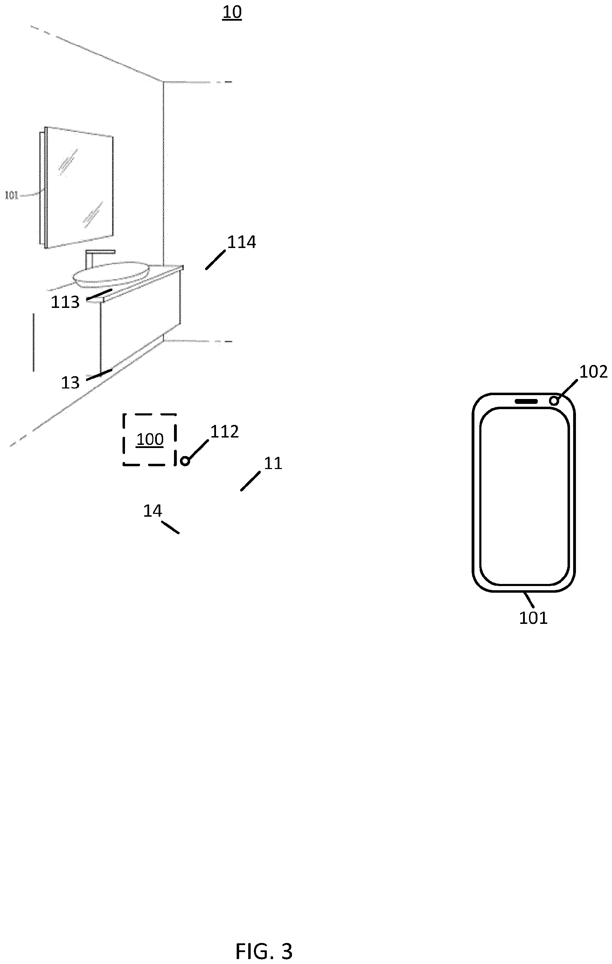

FIG. 3 illustrates another example hygiene reporting system for a bathroom setting 10. The bathroom setting 10 may include a data collection fixture such as a sink including a faucet 11 and a basin 14, and/or a mirror 13. The embodiment of FIG. 3 illustrates a mirror 13 comprising the hygiene controller 100 and at least one sensor 112. The sensor 112 may include a proximity sensor or a camera. The mirror 13 may also include a display 113 and/or a touch surface 114 formed in layers or in a single device. Additional, different, or fewer components may be included.

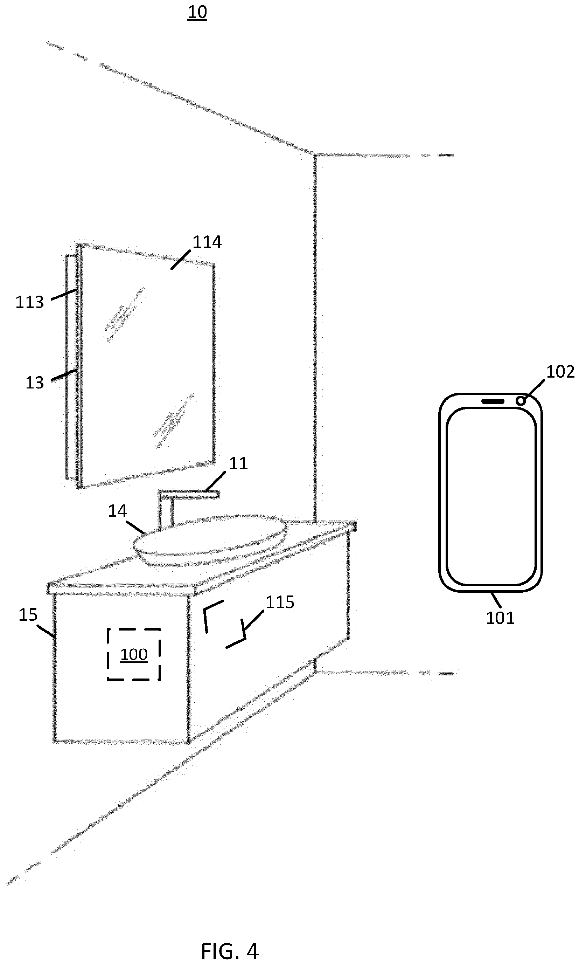

FIG. 4 illustrates another example hygiene reporting system. The bathroom setting 10 may include a data collection fixture such as a sink including a faucet 11 and a basin 14, and/or a mirror 13. The embodiment of FIG. 4 includes a cabinet 15 comprising the hygiene controller 100. The cabinet 15 may also include a mount 115 for supporting the participating endpoint 101. The mount 115 may include a bracket, a grip, or another securing mechanism that is configured to couple the participating endpoint 101 to the cabinet 15. From a fixed position on the cabinet 15, the participating endpoint 101 may collect data from at least one sensor such as the camera 102. Thus, the mount 115 may be positioned based on the position of the participating user. Other positions for the mount 115 such as on the wall or on the mirror 13 are possible. Additional, different, or fewer components may be included.

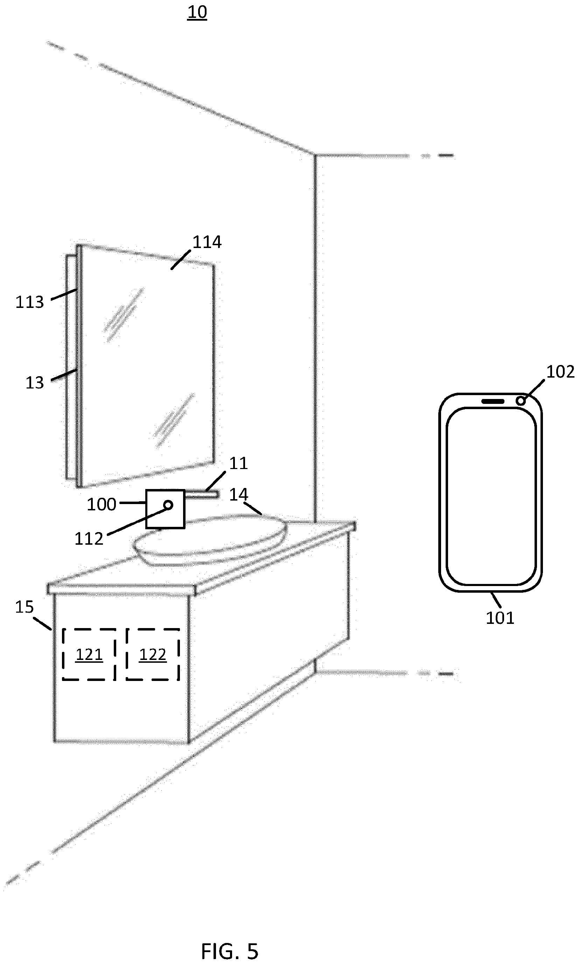

FIG. 5 illustrates another example hygiene reporting system. The bathroom setting 10 may include a data collection fixture such as a sink including a faucet 11 and a basin 14, and/or a mirror 13. The embodiment of FIG. 5 illustrates a faucet 11 including at least one sensor such as a camera 112 or a flow sensor as described in other embodiments. In addition, the faucet 11 may comprise the hygiene controller 100. Thus, the faucet 11 may include a housing enclosing the controller 100 and/or the camera 112 that is water sealed. The controller 100 may also be in communication with a proximity sensor for turning on and off the faucet 11.

In addition, the cabinet may include one or more flow sensors such as water source sensor 121 and a drain sensor 122. The water source sensor 121 detects when, or an amount of, water is dispensed through the faucet 11. The drain sensor 122 detect when, or an amount of, water that is drained from the basin 14. Additional, different, or fewer components may be included.

The hygiene controller 100 may compare the hygiene criteria to the hygiene performance data from the water source sensor 121 and the drain sensor 122. When the hygiene criteria includes whether or not a particular device has been used, the comparison may compare the usage data from the water source sensor 121 or the drain sensor 122 to a water usage value in the hygiene criteria. The hygiene controller 100 may determine whether a basin has been filled with water, or a container has been filled, based on a comparison of the water source sensor 121 and the drain sensor 122. When a flow detected by the drain sensor 122 is substantially less than a flow detected by the water source sensor 121, water has been collected by the basin or another container. The hygiene criteria may indicate that water should be collected for certain activities such as filling a water bottle or washing a face.

The hygiene controller 100 may compare the hygiene criteria for the the water source sensor 121 and the drain sensor 122 to the hygiene performance data in order to calculate a score for the participating user. The score may be a numerical representation of the extent that the hygiene criteria were met by the hygiene performance data.

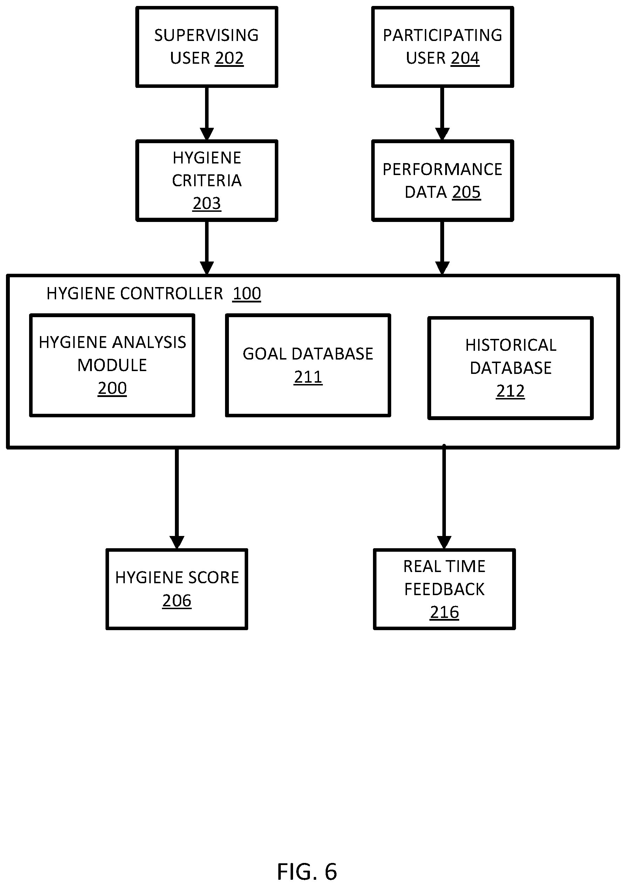

FIG. 6 illustrates example computing devices for the hygiene reporting system. The hygiene controller 100 receives data from two or more users such as a supervising user 202 and a participating user 204. The supervising user 202 provides hygiene criteria 203 as described herein. The participating user 204 performs one or more actions measured by performance data 205. The performance data 205 may describe actions identified from image processing on images or video detected by the camera 112. The performance data 205 may describe actions derived from radar detection of the user.

The hygiene controller 100 may include one or more communication interfaces to communicate with the supervising user 202 (supervising endpoint 111) and a participating user 204 (participating endpoint 101) and any associated sensors. The communication interface is configured to receive at least one hygiene criterion from the supervising endpoint 111 and receive hygiene performance data related to a hygiene activity of the participating user via the participating endpoint 101 or a sensor.

The hygiene controller 100 may include a hygiene analysis module 200 and at least one data storage element including a goal database 211 and a historical database 212. The hygiene analysis module 200 may be configured to perform an analysis of the hygiene performance data 205 according to at least one hygiene criterion 203 and calculate a score for the participating user 204 based on the analysis of the hygiene performance data 205.

The goal database 211 may include hygiene criteria. The hygiene criteria may be associated with predetermined time periods or predetermined users. The supervising user 202 may define the predetermined time period or users when sending the hygiene criteria 203 to the hygiene controller 100.

The hygiene controller 100 may generate a hygiene score 206 and/or real time feedback 216. The historical database 212 may include results of the analysis. The results may include data for the comparison of the hygiene performance data 205 to the hygiene criterion 203. The results may include the hygiene score 206 for the participating user based on the analysis. The results may be stored with a timestamp. Thus, weekly or monthly reports may be generated from the historical database 212 regarding the hygiene practices of the participating user 204 over time.

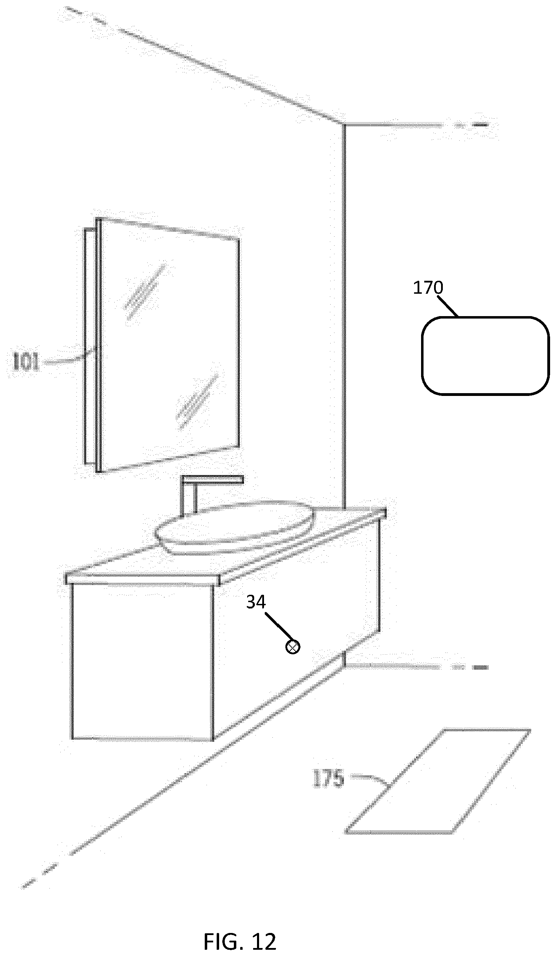

The results may be scored with a user identifier for the participating user 204 and/or the supervising user 202. The identifier may be exchanged between the hygiene controller 100 and the endpoint 101 or endpoint 111. The identifier may be detected based on identification of the user through the sensor data. The identifier may be based on a signature of the user from a characteristic of the user (e.g., weight detected by sensor 155 in FIG. 12). The identifier may be determined based on a login of the participating user 204 and/or the supervising user 202.

The hygiene controller 100 may also provide the hygiene score 206 or the real time feedback 216 back to the supervising user 202 or the participating user 204 in a variety of techniques. The hygiene score 206 may be sent and displayed by the corresponding mobile device (mobile device 101, 111). As described in the following examples, the hygiene score 206 or the real time feedback 216 may be relayed through the mirror 13 or another device in the bathroom setting. The mirror 13 may include an interface associated with the sensor (e.g., camera 112). The hygiene controller 100 detects the hygiene operation at the faucet 11 or another bathroom appliance based on data collected by the sensor and provides real time feedback 216 in response to the detected hygiene operation. The real time feedback 216 may be overlaid on an image in the mirror 13.

Any of the examples may include an alert device (e.g., audio, video, haptic, etc.) configured to alert the supervising user 202 or the participating user 204. Any of the examples may include a display configured to provide real time feedback 216 to the participating user 204.

Other alerts besides those based on the hygiene criteria 203 may be provided. For example, when the flow sensor indicates that continuous water usage exceed a predetermined threshold, the alert may inform the participating user 204 that water should be conserved and the faucet 11 turned off.

In one embodiment, a nonvisual cue may be provided to the participating user 204 in response to the score. The nonvisual cue may include an audio message played by a dedicated speaker or the participating mobile device. The dedicated speaker may be integrated with the cabinet 15 or the mirror 13. The nonvisual cue may include a smell emitted from the cabinet 15 or the mirror 13.

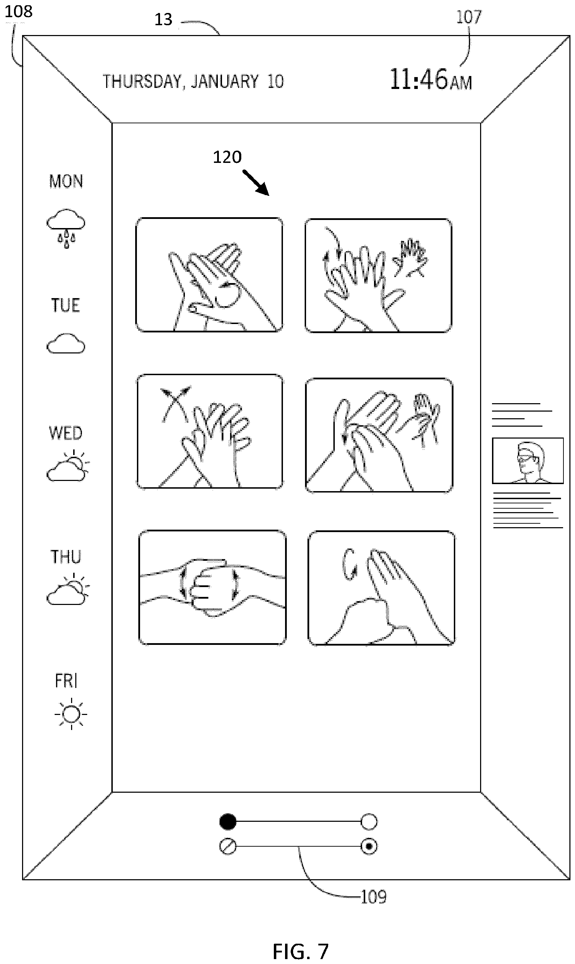

FIG. 7 illustrates an example mirror 13 for the hygiene reporting system. The mirror 13 may include one or more display interfaces or portions such as time panel 107, weather panel 108, and augmented reality display 120. The time panel 107 may display the current time, calendar information, or other selectable data. The weather panel 108 may include the projected weather for the location of the mirror 13.

An input 109 may be configured to adjust display properties for one or more of the display portions of the mirror 13. The input 109 may determine the type of information displayed. The input 109 may determine brightness or color selection for the augmented reality display 120.

The augmented reality display 120 may include at least one graphic that is overlaid on the natural reflection of the mirror 13. The at least one graphic may be based on the at least one hygiene criterion 203 or the comparison of the at least one hygiene criterion 203 to the hygiene performance data 205. In some examples, the at least one graphic may be a sequence of images for hygiene as an animation. As shown in FIG. 7, the at least one graphic may be stages of a handwashing process in different images.

The hygiene controller 100 may compare sensor data (e.g., images collected by the camera 12) to a handwashing template and select one of the handwashing stages to display on the augmented reality display 120. In another example, the hygiene controller 100 may start a timer and highlight or display the images for the handwashing stages in a predetermined order. As described in additional examples herein, other augmented reality options are possible.

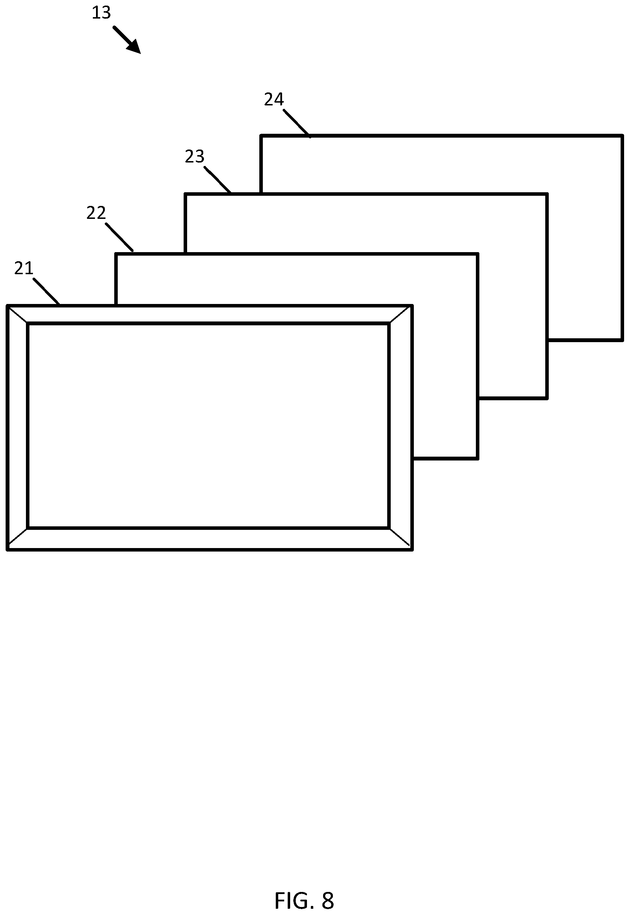

FIG. 8 illustrates an exploded view of another example mirror 13 for the hygiene reporting system. The mirror 13 may include a frame 21, a touch layer 22, a mirror layer 23, and a display layer 24. The frame 21 may include a bezel formed from wood, glass or plastic. Additional, different, or fewer components may be included.

The touch layer 22 may be configured to receive inputs from a user. The touch layer 22 may include a capacitive sensor where a conductive grid having a predetermined electromagnetic field that is manipulated from a user's touch. The touch layer 22 may include infrared beams in at least two directions where disruption of the beams indicate a location of the user's touch.

The mirror layer 23 may be a plastic or glass substrate having a coating to cause the reflection. The reflective surface of the mirror layer 23 may be a two-way mirror so that the display layer 24 is visible through the mirror layer 23. The display layer 24 may include a light emitting diode (LED) or liquid crystal display (LCD). Images from the display layer 24 appear overlaid on the reflection from the mirror layer 23.

In one alternative, the mirror layer 23 is omitted. The mirror instead is simulated as a virtual mirror using images captured by a camera and provided by the display layer 24.

In one alternative, rather than the display layer 24, the augmented reality display 120 is projected onto the mirror layer 23. In other words, the mirror 13 may include a projector configured to generate a light projection that is overlaid on the reflection in the mirror layer 23 to generate the real time feedback 216.

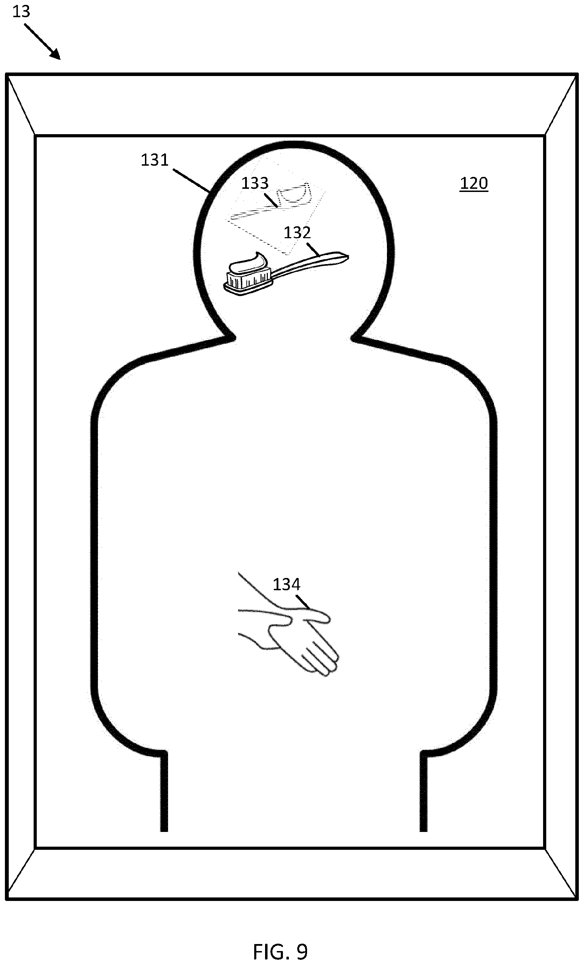

FIG. 9 illustrates another example mirror 13 for the hygiene reporting system using the augmented reality display 120. The augmented reality display 120 may include a silhouette 131, a toothbrushing indicator 132, a flossing indicator 133, and a handwashing indicator 134. Any of these objects may be overlaid on the reflection of the user. The hygiene controller 100 may be configured to select one of the objects in response to the comparison of the hygiene criteria 203 and the hygiene performance data 205. Additional, different, or fewer objects may be used.

In this embodiment, the user may step into the silhouette 131 and align the user's self with the silhouette 131. The objects (e.g., toothbrushing indicator 132, the flossing indicator 133, and/or the handwashing indicator 134) may be in predetermined positions with respect to the silhouette 131. When the user's reflection is aligned with the silhouette 131, the objects may be in predetermined positions with respect to the user's reflection.

The hygiene controller 100 may compare sensor data (e.g., images collected by the camera 12) to templates to determine when particular hygiene events have occurred. The hygiene controller 100 may illuminate all of the objects in response to detection of the user in the presence of the mirror 13. The hygiene controller 100 may update the real time feedback 216 after a hygiene operation or portion thereof is detected. When the hygiene controller 100 determines that a hygiene event has been accomplished, the corresponding object is removed from the augmented reality display 120. For example, when the hygiene controller 100 determines that brushing has occurred, the toothbrushing indicator 132 is removed. When the hygiene controller 100 determines that flossing has occurred, the flossing indicator 133 is removed. When the hygiene controller 100 determines that handwashing has occurred, the handwashing indicator 134 is removed.

As described below, the objects (e.g., toothbrushing indicator 132, the flossing indicator 133, and/or the handwashing indicator 134) may be displayed in a predetermined sequence, each for a predetermined duration.

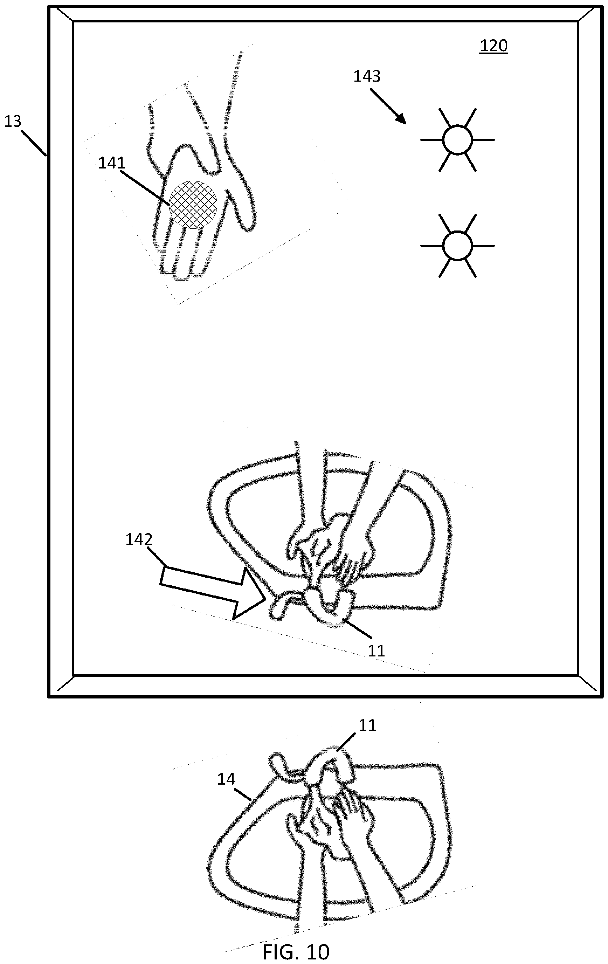

FIG. 10 illustrates another example mirror 13 for the hygiene reporting system and objects overlaid by the augmented reality display 120 on the reflection in the mirror. Example objects overlaid by the augmented reality display 120 include a hygiene indicator such as highlight area 141 or an arrow 142. The positions of these objects in FIG. 10 are for illustration and may not correspond to typical positions. Additional, different, or fewer objects maya be used.

The hygiene controller 100 may compare sensor data (e.g., images collected by the camera 12) to templates or hygiene criteria 203 to determine when particular hygiene events have or have not occurred. The hygiene controller 100 may determine which portions of the user's hands been sufficiently washed or scrubbed based on the sensor data. The hygiene controller 100 generates the highlight area 141 to instruct the user to pay particular attention to a specific portion of the user's hands. The highlight 141 area may be directly overlaid on the reflection of the user's hands in real time.

The hygiene controller 100 may determine the next sequence in the handwashing process. The arrow 142 may indicate to the user to turn on the water, turn off the water, etc. as part of the handwashing process. The arrow 142 may be paired with a textual message (e.g., "turn on"). The arrow 142 may be directly overlaid on the reflection of the faucet 11, basin 14, or surrounding area in real time as it is reflected on a surface of the mirror 13.

Another example object may include a score indicator 143. The score indicator 143 may change color or otherwise flash as the hygiene criteria 203 are met. The score indicator 143 may be displayed in conjunction with the objects overlaid on the reflection of the user and/or bathroom device.

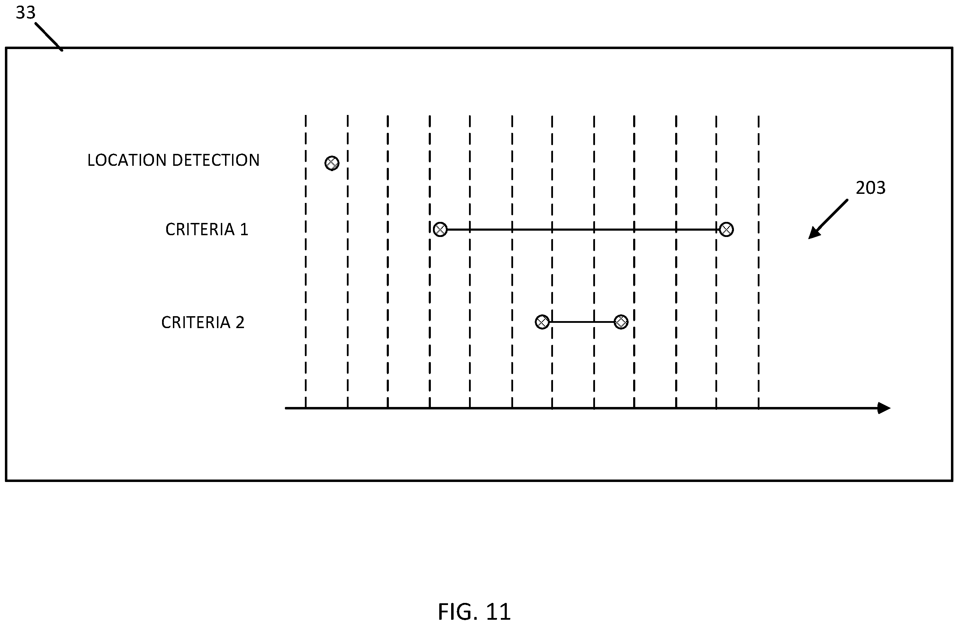

FIG. 11 illustrates a chart 33 an example sequence for hygiene criteria 203. The horizontal axis may represent time. The sequence for the hygiene criteria 203 may be defined by a series of events that occur in time. For example, criterion 1 is met, then criterion 2 is met is an example sequence. The sequence may specify that certain events occur simultaneously or in series in time. The sequence may specify time durations for the events.

One of the events (e.g., the initial event in the sequence) may be detection of the location or presence of the user. Presence or location may be detected in a variety of ways (e.g., analysis of image data from a camera 112, proximity sensor). In addition, FIG. 12 illustrates another example hygiene reporting system including a location sensor 175. The location sensor 175 may be incorporated in the floor or a mat. The location sensor 175 determines whether the user is in the correct position to further evaluate the hygiene criteria. In another example, the location sensor 175 measures the weight of the user and the identity of the user is determined based on weight. The location sensor 175 may include a pressure sensor or a pattern of pressure sensors. The location sensor 175 may be incorporated into a rug, a tile, or a floor. The location sensor 175 may include a light sensor or a proximity sensor. The location sensor 175 may included structured light was a light projector 34 mounted on the cabinet. Alternatively, a camera may be mounted on the camera and collect images at the location shown by the location sensor 175 to detect the presence or identify of the user.

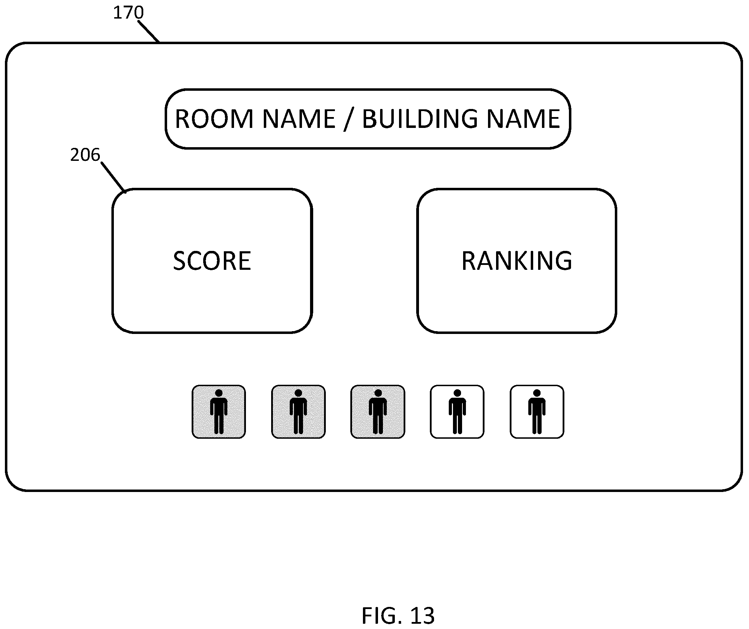

FIG. 13 illustrates an example interface 170 for a hygiene reporting system. FIG. 12 illustrates an example placement of the interface 170. The example interface 170 may provide a location identifier for the bathroom setting 10 such as a room name and/or building name. The interface 170 may report statistics for the bathroom setting 10. In some examples, the statistics are for one participating user 204. A message may be presented by interface 170 or by the mobile device may indicate how the particular user (e.g., the current user) has performed in meeting the hygiene threshold. An example, performance may be 50% compliant or 80% compliant.

Similarly, the message may be presented by interface 170 or by the mobile device may indicate the cumulative performance for the bathroom setting 10. In some examples, the statistics apply to multiple participating users. The statistic may include a ranking for the location as compared to other locations such as a ranking within a company, a building, or an institution. For example, this location is ranked 3 out of 12 most hygiene compliant. Another icon may describe how many of the recent set of users have compliant with the hygiene criteria.

The data indicative of the hygiene score 206 may be stored across time periods. For example, the data may be tracked for the user (e.g., identifier of mobile device) or for the bathroom setting 10. The hygiene controller 100 may determine hygiene performance across the time period. The controller 100 may determine how often the particular user meets the hygiene threshold, which may be the number of times the hygiene threshold is met divided by (or in ratio to) the total number of times the user was present in the bathroom setting 10 or any lavatory setting. The controller 100 may determine how often the particular user meets the hygiene threshold in a particular day, week, month or other time period.

The controller 100 may determine how often a group of users meet the hygiene threshold, which may be the number of times the hygiene threshold is met divided by (or in ratio to) the total number of times any of the group of users were present in the bathroom setting 10 or any lavatory setting. The controller 100 may determine how often the group of users meets the hygiene threshold in a particular day, week, month or other time period.

The controller 100 may determine how often all users in the bathroom setting 10 meets the hygiene criteria 203, which may be the number of times the hygiene criteria 203 are met divided by (or in ratio to) the total number of times any users were present in the bathroom setting 10. The controller 100 may determine how often users of the particular bathroom setting 10 met the hygiene criteria 203 in a particular day, week, month or other time period.

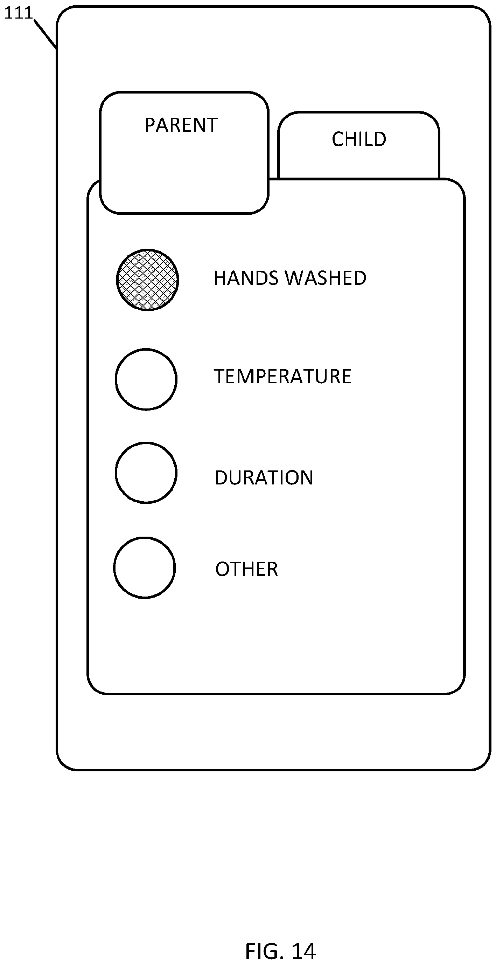

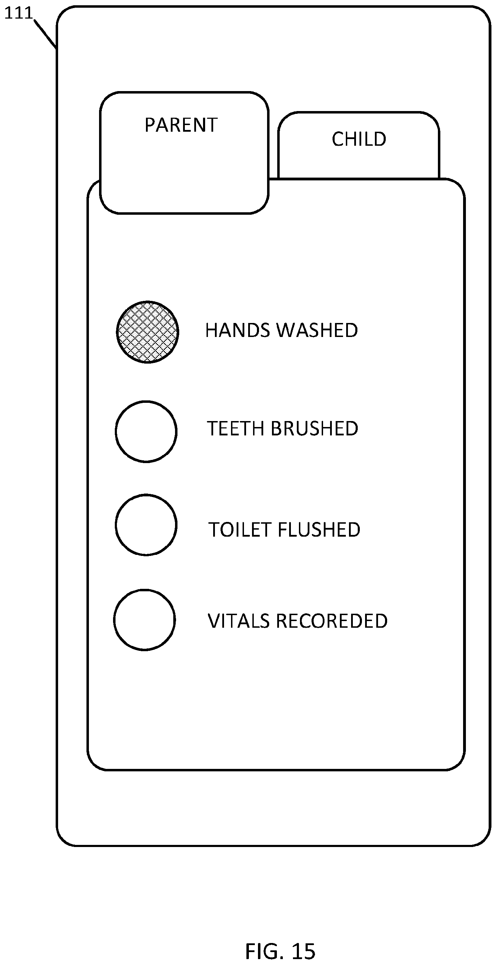

FIGS. 14 and 15 illustrate example supervising interfaces for the hygiene reporting system. In FIGS. 14 and 15, tabs are provided for the parent and child. The parent tab indicates the hygiene events or hygiene criteria 206 that have been accomplished by the child. The child tab may track the information provided by the participant endpoint 101. In FIG. 14, the hygiene criteria 203 are related to a single event and includes indicators for criteria of temperature and duration as well as an overall indicator for the event (e.g., hands washed). In FIG. 15, the hygiene criteria are related to different events (e.g., hands washed, teeth brushed, toilet flushed, and vitals recorded).

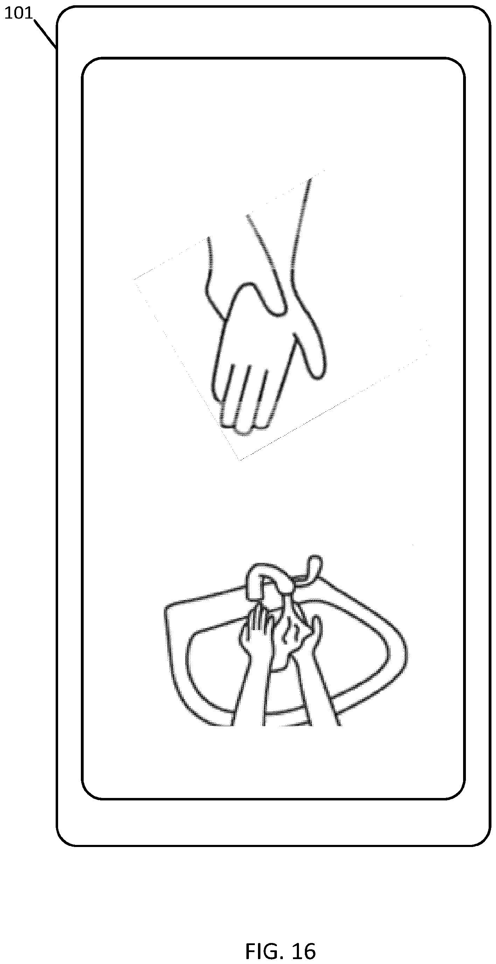

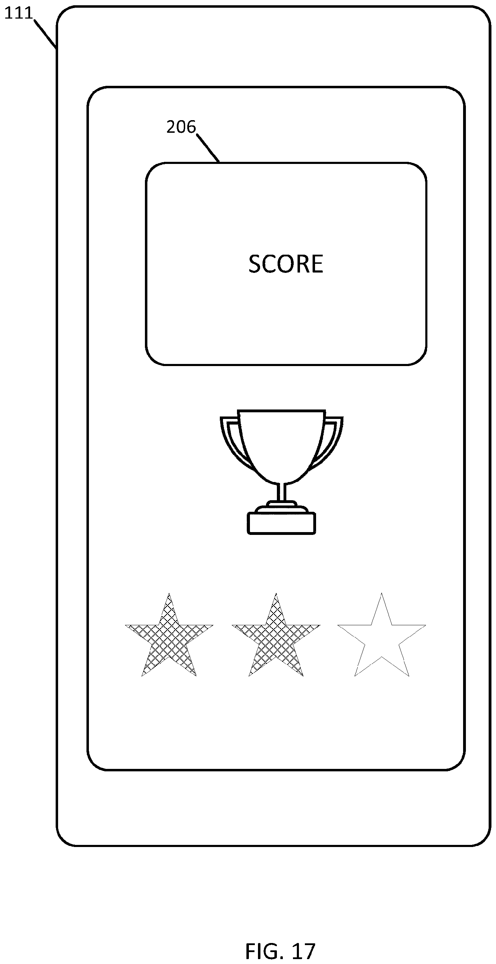

FIGS. 16 and 17 illustrate examples of the participating interface 101 for the hygiene reporting system. In FIG. 16, images of the user's hands or bathroom device are displayed as collected by the camera 12. One or more objects or indicia may be overlaid on the user's hands or bathroom device to provide instructions or indicate progress with the hygiene event. In FIG. 17, the hygiene score 206 is displayed. In addition, visual cues or rewards may be provided in response to the hygiene score 206 when the user is in proximity to the hygiene activity. These visual cues or rewards (e.g., star rating or a trophy) may be combined with the hygiene score 206.

In addition or alternatively, the participating endpoint (interface) 101 may provide an incentive to the participating user 204 in response to the score. In other words, when the hygiene score 206 exceeds a predetermined value, the participating endpoint (interface) 101 rewards the participating user 204. The incentive may be monetary. The incentive may be access to a virtual object or time with an application or game on the mobile device. The incentive may be accessed based on personal preferences or assignments for a particular user, which are selected based on an identifier for the participating user 204.

In addition or in the alternative, the subject matter of FIGS. 14-17 may supplement a virtual world, video game, mobile application, or environment in the metaverse, which may be referred to collectively or individually as a hygiene incentive application, that is tied to the hygiene controller 100 and the comparison of at least one hygiene criterion to hygiene performance data. The hygiene criteria may specify a volume of water used or a time duration that water is used to incentivize water conservation.

In one example, the participating user 204 and/or participating endpoint 101 defines one or more characters, avatars, or accounts for the hygiene incentive application. As described in any of the embodiments herein, the hygiene controller 100 monitors or evaluates the hygiene activity of the user in the real world, and in response, provides a reward, an object, or other inventive in the hygiene incentive application. For example, when the hygiene performance data indicates that the hygiene criteria are met (e.g., water is being conserved and the task is being performed), the hygiene controller 100 may instruct the participating endpoint 101 to provide an upgrade to a character. The upgrade may be implemented as an award or indicator in the game (e.g., FIG. 17).

For example, when the hygiene performance data indicates that the hygiene criteria are met (e.g., water is being conserved and the task is being performed), the hygiene controller 100 may instruct the participating endpoint 101 to provide in-game money or points that can be used to redeem an in-game object (e.g., a feature, tool, or device used in the game).

Additional incentives or points may be provided to the character for participating is certain activities in the hygiene incentive application. The activities may include watching a video, performing a class or other education activity, or signing up for a hygiene challenge.

The hygiene incentive application may include instructional videos displayed at the participating endpoint 101 that instruct the user to perform the hygiene activity. An example video may instruct proper technique for washing hands or brushing teeth. The video may be integrated into game play in the hygiene incentive application.

The hygiene incentive application may include a hygiene log displayed at the participating endpoint 101. Entries in the hygiene log may be populated automatically through the analysis of sensor data in any of the examples above. Entries in the hygiene log may be populated manually. For example, upon completing or attempting a task, the participating user 204 may create an entry in the hygiene log. In response to approval of the hygiene task, the supervising user 202 may approve the task in the hygiene log. In response, to approval, the hygiene controller 100 may provide any of the incentives described herein.

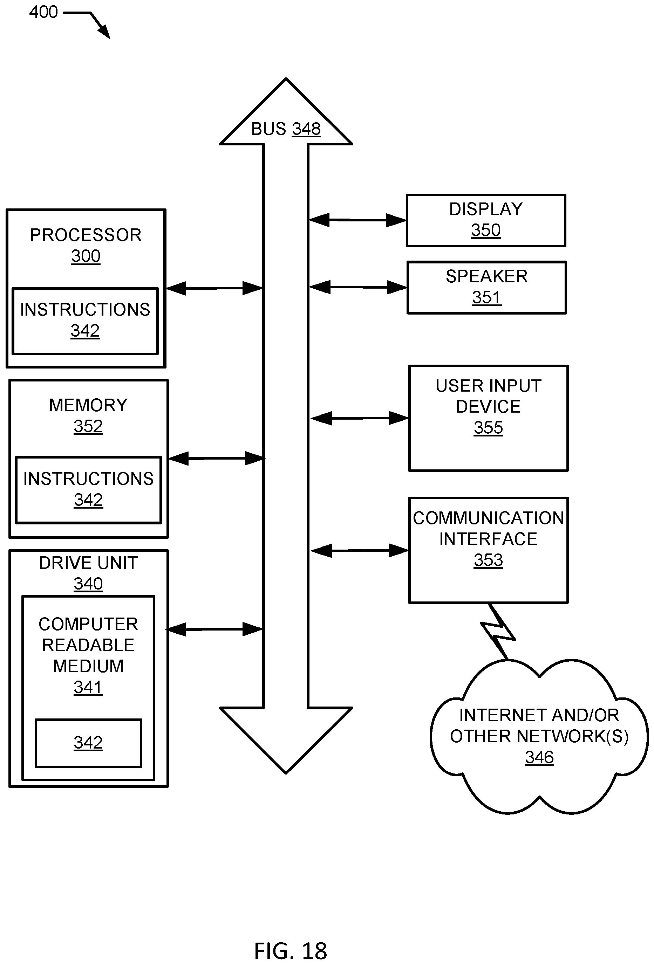

FIG. 18 illustrates example details for the control system 400 for implementing the hygiene controller 100. The control system 400 may include a processor 300, a memory 352, and a communication interface 353 for interfacing with devices or to the internet and/or other networks 346. In addition to the communication interface 353, a sensor interface may be configured to receive data from the sensors described herein. The components of the control system 400 may communicate using bus 348. The control system 400 may be connected to a workstation or another external device (e.g., control panel) and/or a database for receiving user inputs, system characteristics, and any of the values described herein.

Optionally, the control system 400 may include an input device 355 and/or a sensing circuit in communication with any of the sensors. The sensing circuit receives sensor measurements from as described above. The input device 355 may include a touchscreen coupled to or integrated with the mobile device, the mirror, a keyboard, a microphone for voice inputs, a camera for gesture inputs, and/or another mechanism.

Optionally, the control system 400 may include a drive unit 340 for receiving and reading non-transitory computer media 341 having instructions 342. Additional, different, or fewer components may be included. The processor 300 is configured to perform instructions 342 stored in memory 352 for executing the algorithms described herein. A display 350 may be supported by the mirror frame. The display 350 may be combined with the user input device 355.

The control system 400 may include a server that is in communication but remote from the mobile devices (e.g., participating endpoint 101 and the supervising endpoint 111). According to any of the examples herein, the server may be configured to perform an analysis the hygiene performance data 205 according to the at least one hygiene criterion 203 and calculate a hygiene score 206 for the participating user based on the analysis of the hygiene performance data 205.

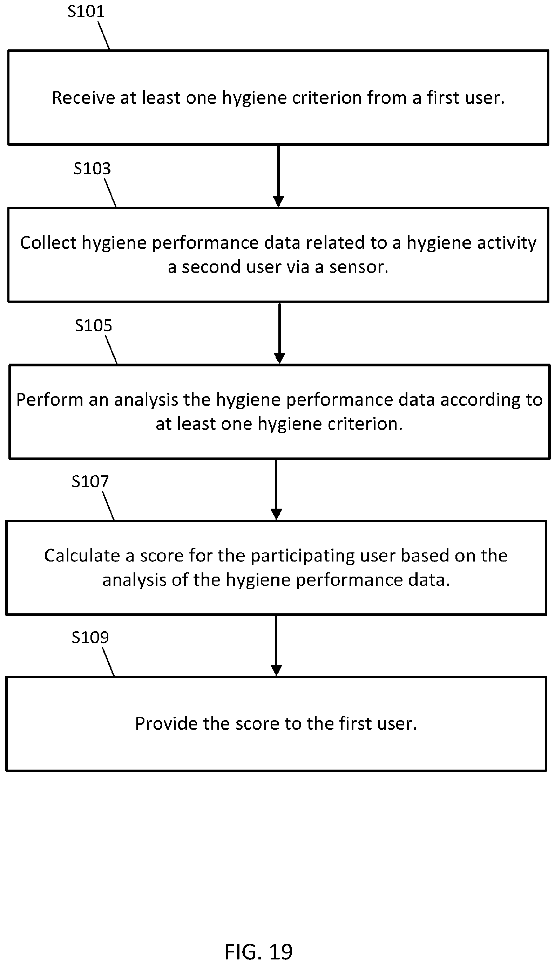

FIG. 19 illustrates a flow chart for the hygiene reporting system or specifically hygiene controller 100. The acts of the flow chart may be performed by any combination of hygiene controller 100, the network device or the server. Portions of one or more acts may be performed by the appliance. Additional, different of fewer acts may be included.

At act S101, the controller 400 (e.g., through communication interface 353) receives at least one hygiene criterion at a mobile device associated with a supervising user. The hygiene criteria may be selected from a list or otherwise defined by the mobile device. The hygiene criteria may include a time value for the hygiene activity of the participating user, a sequence of events for the hygiene activity of the participating user, or a schedule for the hygiene activity of the participating user.

At act S103, the controller 400 receives (directly or indirectly collects) hygiene performance data related to a hygiene activity a participating user via a sensor. In some examples, the controller 400 and sensor are integrated in the same device. In some examples, the sensor data is relayed to the controller 400 through the network.

At act S105, the controller 400 performs an analysis the hygiene performance data according to at least one hygiene criterion. Various embodiments are possible for the comparison of the hygiene performance data to the hygiene criteria. In some examples, the comparison determines whether a particular device has been operated by the user. In some examples, the comparison determines whether the user has moved in an anticipated pattern. In some examples, the comparison confirms a sequence of motion patterns and/or device operation.

At act S107, the controller 400 calculates a score for the participating user based on the analysis of the hygiene performance data. The score may be proportional to the degree of similarity between the hygiene performance data to the hygiene criteria. When multiple criteria are used, the score may be indicative of the number of criteria that are satisfied.

In other embodiments, in addition or in the alternative to the score, the controller 400 generates a message for the participating user based on the analysis of the hygiene performance data. The message may be presented by display 350 or by the mobile device. The command may instruct another device to generate an indicator (e.g., light, audio, vibration). In addition, the controller 400 may store satisfaction of the hygiene criteria in memory 352. For example, the controller 400 may set a flag or other alphanumeric value in a table as data indicative of satisfaction of the hygiene criteria in association with the identifier for the mobile device. The data indicative of satisfaction of the hygiene criteria may be associated with the bathroom setting 10 or a particular lavatory.

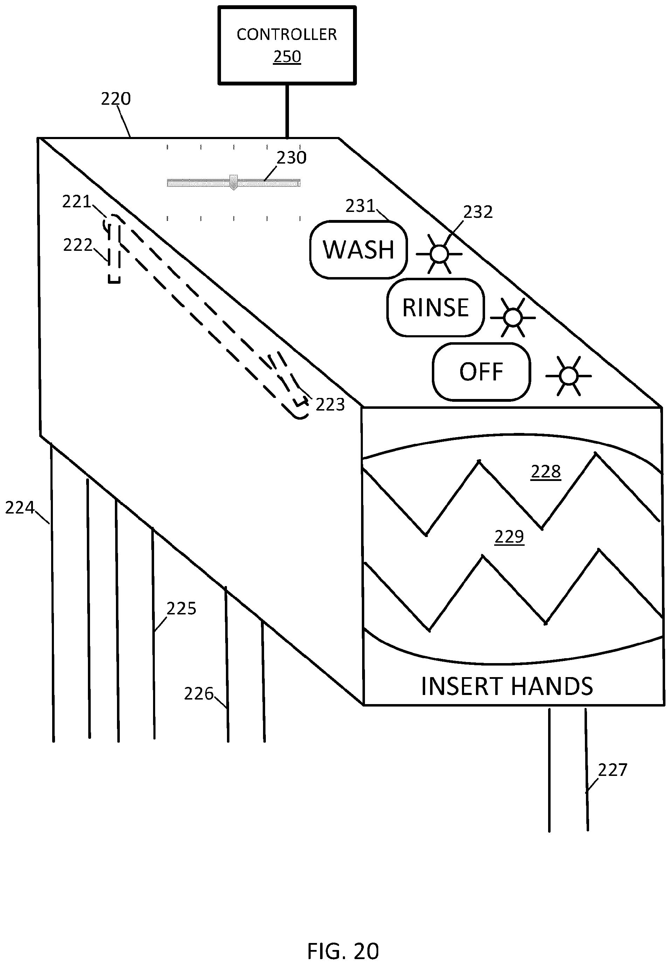

FIG. 20 illustrates an example automated handwashing system shown as a hand washing enclosure 220. The hand washing enclosure 220 may include internal components, shown as dashed lines, that facilitate or automate handwashing within the hand washing enclosure 220. The hand washing enclosure 220 may including plumbing fixtures for the introduction and removal of water and/or cleaning agents from the hand washing enclosure 220. The hand washing enclosure 220 may include user inputs to control the hand washing process. The hand washing enclosure 220 may include indicia to indicate settings for the hand washing process. Additional, different or fewer components may be included.

The hand washing enclosure 220 may include an opening 229 configured for the insertion of one or more hands of a user. The opening 229 may be defined according to a flap 228 formed of rubber or foam. The flap 228 is flexible, which allows the user to increase the size of the opening 229 as needed.

The hand washing enclosure 220 may include at least one input fixture configured to introduce one or more fluids to the hand washing enclosure. The input fixtures may include a plurality of water input pipes (e.g., hot water input 224 and cold water input 225) configured to introduce water to the hand washing enclosure 220. The input fixtures may include a soap input tube 226 configured to introduce soap to the hand washing enclosure 220.

The handwashing enclosure 220 may include a drain 227 configured to remove the one or more fluids from the hand washing enclosure 220.

The hand washing enclosure 220 may include at least one mobile washing tool configured to wash hands that are inserted into the hand washing enclosure 220. The mobile washing tool may contact the hands. Example contact mobile washing tools include a brush, a sponge, or a cloth. The mobile washing tool may be separated from the hands. Example non-contact mobile washing tools may include a high pressure sprayer.

The hand washing enclosure 220 may include a conveyor 221 configured to move the at least one mobile washing tool into proximity with the one or more hands of the user. The conveyor 221 may support the mobile washing tool in a first orientation (shown by dotted lines 222) when engaging the hands and/or in motion from a first position to a second position. The conveyor 221 may support the mobile washing tool in a second orientation (shown by dotted lines 223) when returning the mobile washing tool from the second position to the first position.

The hand washing enclosure 220 may include at least one visual indicator configured to indicate a stage or mode of the hand washing enclosure. The visual indicator may be responsive to operation of the hand washing enclosure 220. The visual indicator may include a light 232 or a window 231 including text that is displayed on a screen or otherwise illuminated. The light 232 or the window 231 may communicate a mode selection, an activation for the hand washing enclosure, a temperature setting for the hand washing enclosure, or a duration for the hand washing enclosure. The mode selection may be the current mode such as wash cycle, rinse cycle, or dry cycle. The activation for the hand washing enclosure 220 may indicate whether the device is in operation or not. The temperature setting for the hand washing enclosure 220 may define a temperature or a ratio to mix water from the hot water input 224 and cold water input 225. The duration for the hand washing enclosure may be a time remaining in the hand washing cycle.

The hand washing enclosure 220 may include a mobile drying tool configured to direct air to the one or more hands of the user. A fan may direct air through the mobile drying tool. The conveyor 221 may be configured to move the mobile drying tool from the first position to the second position and vice versa. The conveyor 221 is configured to move the mobile drying tool into proximity with the one or more hands of the user.

The hand washing enclosure 220 may include a controller 250. The controller may send commands to a motor for the conveyor 221, one or more valves for the hot water input 224, the cold water input 225, the soap input tube 226, and/or the drain 227. The controller 250 may include a timer. In one example, time is measured from the insertion of one or more hands of a user (e.g., as detected by a proximity sensor or motion sensor at the opening 229). The controller 250 may open a valve for the at least one input fixture in response to the time elapsed from the insertion of one or more hands of a user. The controller 250 may open a valve for the hot water input 224, the cold water input 225, the soap input tube 226, and/or the drain 227 inn sequence each for a predetermined amount of time.

The actuation or advancement of the conveyor 221 may also be based on the elapsed time of the timer. The controller 250 may actuate the conveyer in response to the time elapsed from the insertion of one or more hands of a user. The controller 250 may operate a motor (e.g., stepper motor) in response to the timer. The motor may cause the conveyor 221 to move the at least one mobile washing tool into contact or proximity of the user's hands. The controller 250 may cause the conveyor 221 to move multiple tools (e.g., scrubbing tool, rinsing tool, drying tool) is succession, each starting and/or ending at a predetermined elapsed time.

The hand washing enclosure 220 may include a user input device 230 configured to receive a command from the user for operation of the hand washing enclosure. The command from the user includes a mode selection, an activation for the hand washing enclosure, a temperature setting for the hand washing enclosure, or a duration for the hand washing enclosure.

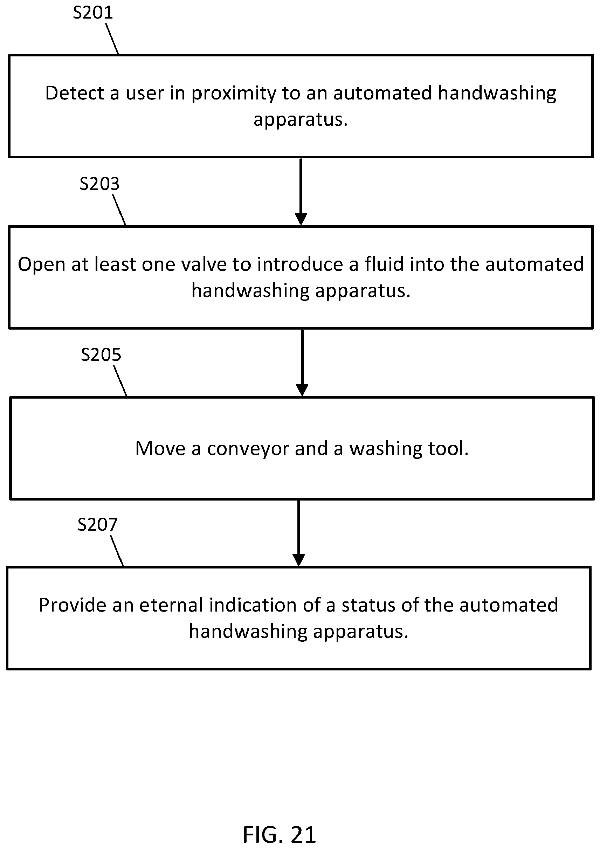

FIG. 21 illustrates a flow chart for the automated handwashing system. In this example, the controller 250 may be implemented as controller 400, as shown in FIG. 18, may perform the acts of the flow chart. Additional, different of fewer acts may be included.

At act S201, the controller 400 detects a user in proximity to an automated handwashing apparatus. One or more hands of a user may be detected within the hand washing enclosure 220 or near the hand washing enclosure.

At act S203, the controller 400 opens at least one valve to introduce a fluid into the automated handwashing apparatus in response to the detection of the user's hand. The at least one valve may be associated with one or more of at least one water input pipe configured to introduce water to the hand washing enclosure and a soap input tube configured to introduce soap to the hand washing enclosure. In some examples, opening the valve may be in response to receipt of a command from the user for operation of the hand washing enclosure.

At act S205, the controller 400 actuates a motor, solenoid, or other driving mechanism to advance a conveyor to move at least one mobile washing tool into proximity with the one or more hands of the user.

At act S207, the controller 400 provides an indication of a status of the hand washing apparatus. For example, the controller 400 may Illuminate at least one visual indicator configured to indicate a stage or mode of the hand washing enclosure.

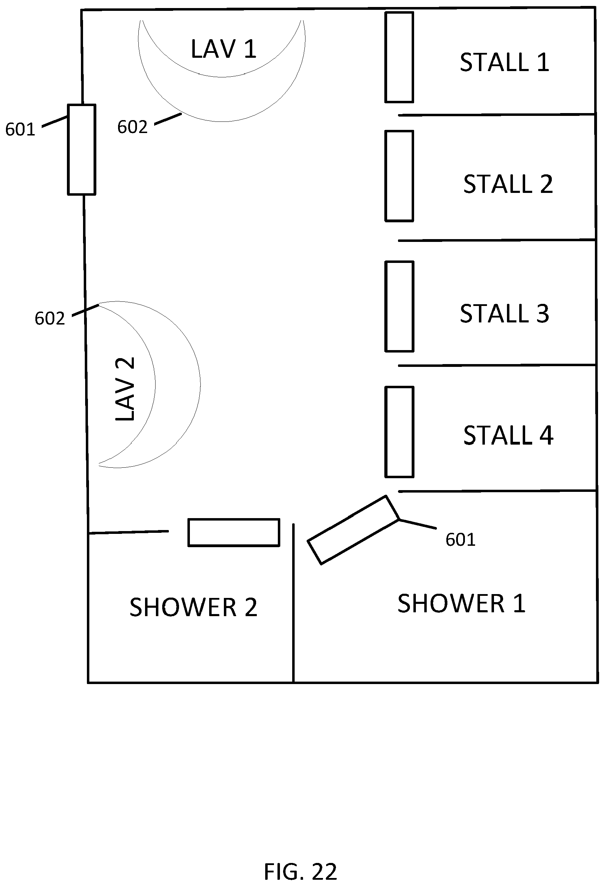

FIG. 22 illustrates an example air current system configured to create hygienic air barriers or air curtains in a bathroom setting. The air current system includes a plurality of air barriers which may have a cross section that is substantially rectangular (e.g., air curtains 601) or substantially curved or crescent shaped (e.g., air curtains 602). The air barriers generally extend vertically from the ceiling to the floor and provide a barrier for particles or aerosols in a horizontal direction or substantially in a horizontal direction. The flow of air between a first space and a second space is effective in block particle or aerosols from traveling between the first space and the second space. The barrier, however, is only air, which allows people or object to freely travel between the first space and the second space without the need of removing the barrier, opening the barrier, or turning off the air flow.

Each of the air curtains may be positioned to isolate a bathroom device from its surroundings. Each of the illustrated stalls includes an air curtain 601 to isolate a toilet or urinal from the rest of the bathroom. Each of the showers includes an air curtain 601 to isolate the shower from the rest of the bathroom. Each of the illustrated lavatories includes an air curtain 602 to isolate the lavatory from the rest of the bathroom.

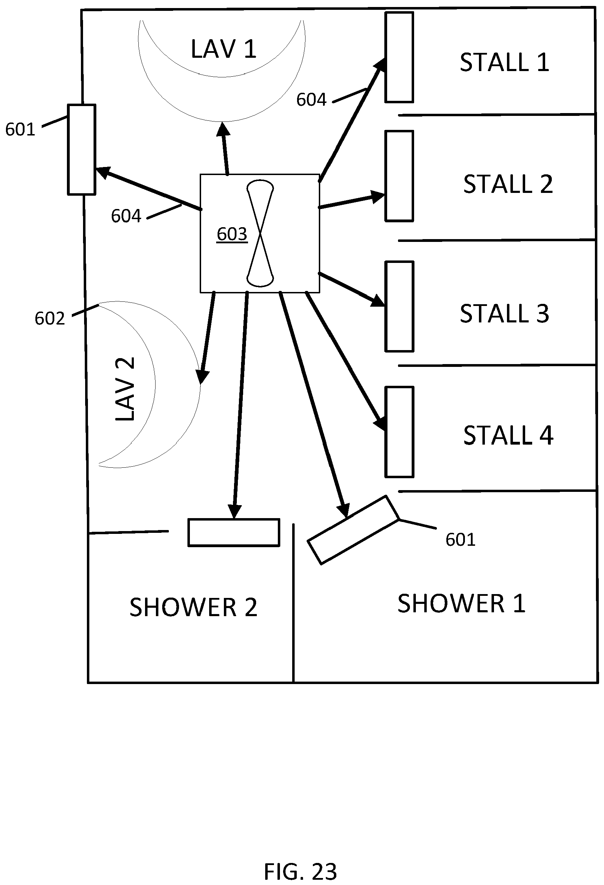

FIG. 23 illustrates another view of the air current system including a fan 603 configured to create an air flow and a duct 604 configured to accelerate and direct the air flow as an air curtain to isolate a bathroom device from surroundings of the bathroom device. The duct 604 may include a tapered portion that reduces the cross section off the air path in order accelerate the air flow speed. A distribution assembly between the duct 604 and the fan 603 may be configured to divide and direct the air flow into multiple paths. At the end of each of the duct, an air curtain outputs an air curtain associated with respective bathroom devices.

At a lower position than the fan 603, such as at the floor, a vacuum device may be aligned with the air curtain and configured to exhaust the air current away from the surroundings of the bathroom device.

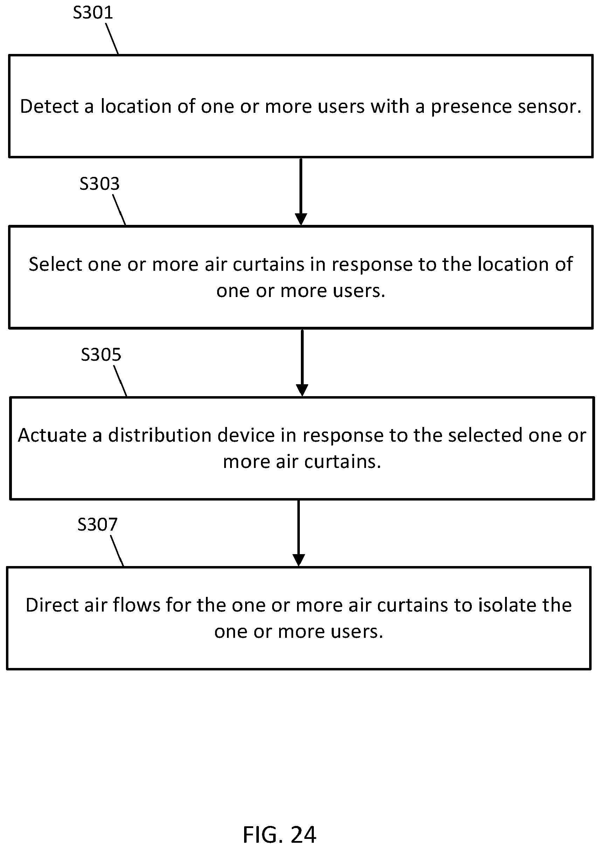

FIG. 24 illustrates a flow chart for the air curtain system. In this example, the controller, implemented as controller 400, as shown in FIG. 18, may perform the acts of the flow chart. Additional, different of fewer acts may be included.

At act S301, the controller 400 may receive data from one or more presence sensors. The presence sensors may be arranged in a grid to detect users at various locations in the floor plan of FIGS. 22 and 23. The controller 400 determines the locations of one or more users based on the sensor data from the presence sensors.

At act S303, the controller 400 selects one or more air curtains in response to the location of one or more users. The air curtains may be selected in order to isolate the users from each other or to isolate one or more users from a bathroom device.

At act S305, the controller 400 generates a command to actuate a distribution device in response to the selected one or more air curtains. The distribution device includes one or more air valves or louvres that directs air through ducts or other pathways.

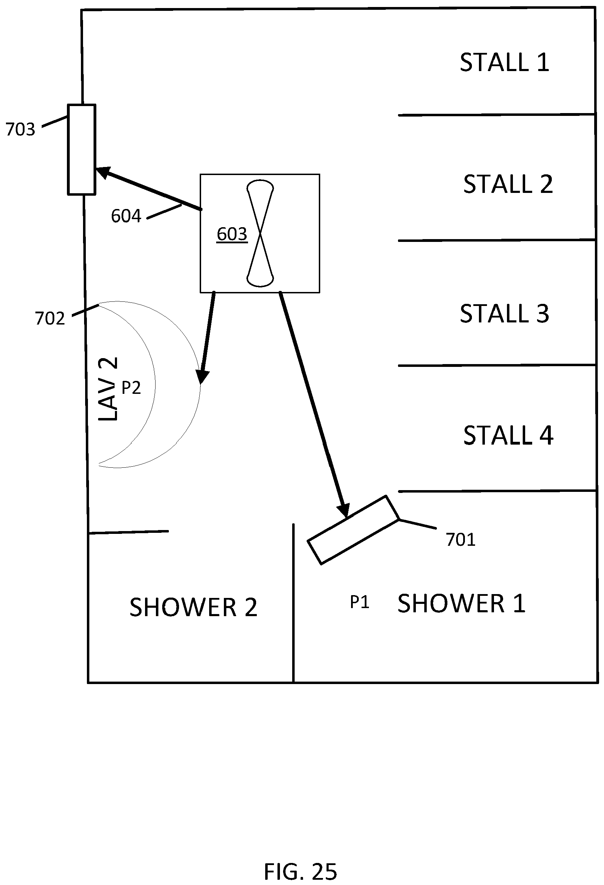

At act S307, directing air flows for the one or more air curtains to isolate the one or more users. FIG. 25 illustrates an example bathroom setting including two users. Person P 1 is in shower 1 and person P2 at Lavatory 2. The presence sensors detect the locations of P1 and P2. Controller 400 selects the distribution device to provide air curtain 701 to isolate person P1 from the rest of the room and air curtain 702 to isolate person P2 from the rest of the room. In addition, air curtain 703 may be selected to provide a barrier at the entrance/exit of the room.

Processor 300 may be a general purpose or specific purpose processor, an application specific integrated circuit (ASIC), one or more programmable logic controllers (PLCs), one or more field programmable gate arrays (FPGAs), a group of processing components, or other suitable processing components. Processor 300 is configured to execute computer code or instructions stored in memory 352 or received from other computer readable media (e.g., embedded flash memory, local hard disk storage, local ROM, network storage, a remote server, etc.). The processor 300 may be a single device or combinations of devices, such as associated with a network, distributed processing, or cloud computing.

Memory 352 may include one or more devices (e.g., memory units, memory devices, storage devices, etc.) for storing data and/or computer code for completing and/or facilitating the various processes described in the present disclosure. Memory 352 may include random access memory (RAM), read-only memory (ROM), hard drive storage, temporary storage, non-volatile memory, flash memory, optical memory, or any other suitable memory for storing software objects and/or computer instructions. Memory 352 may include database components, object code components, script components, or any other type of information structure for supporting the various activities and information structures described in the present disclosure. Memory 352 may be communicably connected to processor 300 via a processing circuit and may include computer code for executing (e.g., by processor 300) one or more processes described herein. For example, memory 298 may include graphics, web pages, HTML files, XML files, script code, shower configuration files, or other resources for use in generating graphical user interfaces for display and/or for use in interpreting user interface inputs to make command, control, or communication decisions.

In addition to ingress ports and egress ports, the communication interface 353 may include any operable connection. An operable connection may be one in which signals, physical communications, and/or logical communications may be sent and/or received. An operable connection may include a physical interface, an electrical interface, and/or a data interface. The communication interface 353 may be connected to a network. The network may include wired networks (e.g., Ethernet), wireless networks, or combinations thereof. The wireless network may be a cellular telephone network, an 802.11, 802.16, 802.20, or WiMax network, a Bluetooth pairing of devices, or a Bluetooth mesh network. Further, the network may be a public network, such as the Internet, a private network, such as an intranet, or combinations thereof, and may utilize a variety of networking protocols now available or later developed including, but not limited to TCP/IP based networking protocols.

While the computer-readable medium (e.g., memory 352) is shown to be a single medium, the term "computer-readable medium" includes a single medium or multiple media, such as a centralized or distributed database, and/or associated caches and servers that store one or more sets of instructions. The term "computer-readable medium" shall also include any medium that is capable of storing, encoding, or carrying a set of instructions for execution by a processor or that cause a computer system to perform any one or more of the methods or operations disclosed herein.

In a particular non-limiting, exemplary embodiment, the computer-readable medium can include a solid-state memory such as a memory card or other package that houses one or more non-volatile read-only memories. Further, the computer-readable medium can be a random access memory or other volatile re-writable memory. Additionally, the computer-readable medium can include a magnetooptical or optical medium, such as a disk or tapes or other storage device to capture carrier wave signals such as a signal communicated over a transmission medium. A digital file attachment to an e-mail or other self-contained information archive or set of archives may be considered a distribution medium that is a tangible storage medium. Accordingly, the disclosure is considered to include any one or more of a computer-readable medium or a distribution medium and other equivalents and successor media, in which data or instructions may be stored. The computer-readable medium may be non-transitory, which includes all tangible computer-readable media.

In an alternative embodiment, dedicated hardware implementations, such as application specific integrated circuits, programmable logic arrays and other hardware devices, can be constructed to implement one or more of the methods described herein. Applications that may include the apparatus and systems of various embodiments can broadly include a variety of electronic and computer systems. One or more embodiments described herein may implement functions using two or more specific interconnected hardware modules or devices with related control and data signals that can be communicated between and through the modules, or as portions of an application-specific integrated circuit. Accordingly, the present system encompasses software, firmware, and hardware implementations.

An aspect provides a method for monitoring hygiene, the method comprising:
receiving at least one hygiene criterion at a mobile device associated with a supervising user;
collecting hygiene performance data related to a hygiene activity a participating user via a sensor;
performing an analysis of the hygiene performance data according to at least one hygiene criterion; and
calculating a score for the participating user based on the analysis of the hygiene performance data.

The method may comprise: providing the score to the mobile device associated with the supervising user.

The method may comprise: providing the score to an interface associated with the sensor.

The interface may be associated with a mirror.

The interface may include augmented reality comprising a reflection in the mirror and at least one graphic for the at least one hygiene criterion displayed in association with the reflection in the mirror.

The method may comprise: providing an incentive to the participating user in response to the score.

The incentive may include a visual cue in proximity to the hygiene activity.

In an implementation, the mobile device for the supervising user may be a first mobile device, and the incentive may include an access time for the participating user with a second mobile device.

The method may comprise: providing a nonvisual cue to the participating user in response to the score.

The nonvisual cue may include an audio message or a scent.

The at least one hygiene criterion may include a time value for the hygiene activity of the participating user.

The at least one hygiene criterion may include a sequence of events for the hygiene activity of the participating user.

The at least one hygiene criterion may include a schedule for the hygiene activity of the participating user.

The method may comprise: receiving at least one weight from the mobile device associated with the supervising user, wherein the score is calculated based on the at least one weight.

The method may comprise: determining an identifier for the participating user; and storing the score with the identifier for the participating user.

Another aspect provides a system for monitoring hygiene, the system comprising:
a first mobile device associated with a supervising user, the first mobile device configured to receive at least one hygiene criterion;
a second mobile device associated with a participating user, the second mobile device configured to collect hygiene performance data related to a hygiene activity of the participating user; and
a server configured to perform an analysis the hygiene performance data according to the at least one hygiene criterion and calculate a score for the participating user based on the analysis of the hygiene performance data.

Another aspect provides an apparatus for monitoring hygiene, the apparatus comprising:
a communication interface configured to receive at least one hygiene criterion from a mobile device associated with a supervising user and receive hygiene performance data related to a hygiene activity a participating user via a sensor; and
a controller configured to perform an analysis the hygiene performance data according to at least one hygiene criterion and calculate a score for the participating user based on the analysis of the hygiene performance data.

The at least one hygiene criterion may include a time value for the hygiene activity of the participating user, a sequence of events for the hygiene activity of the participating user, or a schedule for the hygiene activity of the participating user.

The controller may be configured to identify the participating user and select an incentive based on the score and the participating user.

The apparatus may be a server in communication with the mobile device associated with the supervising user.

Another aspect provides a method for real time feedback for personal hygiene, the method comprising:
detecting a hygiene operation at a bathroom appliance with at least one sensor; and
providing real time feedback in response to the detected hygiene operation, wherein the real time feedback is overlaid on an image of the bathroom appliance.

The image of the bathroom appliance may be a reflection that is reflected on a surface of a mirror.

The image of the bathroom appliance may be a virtual mirror captured by a camera and provided by a display.

The bathroom appliance may include a faucet.

The real time feedback may include an animation.

The real time feedback may include a pointer for a portion of a surface of a hand.

The real time feedback may include a message for the hygiene operation.

In an implementation, wherein the hygiene operation is a first hygiene operation, the method may comprise: detecting a second hygiene operation at the bathroom appliance with the at least one sensor; and updating the real time feedback in response to the detected second hygiene operation.

The real time feedback may include a score in response to the detected hygiene operation.

The score may be a time value for the hygiene operation, a sequence of events for the hygiene operation, or a schedule for the hygiene operation.

The at least one sensor may include a camera and a flow sensor and the detected hygiene operation may be based on user movement detected by the camera and water usage detected by the flow sensor.

Another aspect provides an apparatus for real time feedback for personal hygiene, the apparatus comprising:
a sensor configured to detect a hygiene operation at a bathroom appliance; and
a display configured to provide real time feedback in response to the detected hygiene operation, wherein the real time feedback is overlaid on an image of the bathroom appliance.

The image of the bathroom appliance may be a reflection that is reflected on a surface of a mirror.

The image of the bathroom appliance may be provided by the display.

The apparatus may comprise: a projector configured to generate the real time feedback for the display.

The sensor may include a camera and the detected hygiene operation may be based on user movement detected by the camera.

The sensor may include a flow sensor and the detected hygiene operation may be based on water usage detected by the flow sensor.

The real time feedback may be overlaid on an image of a user.

Another aspect provides a mirror comprising:
a reflective surface;
a sensor configured to detect a hygiene operation of a user at a bathroom appliance; and
a display configured to provide real time feedback in response to the detected hygiene operation, wherein the real time feedback is overlaid on an image of the bathroom appliance or the user provided by the reflective surface.

The reflective surface may be a two-way mirror.

Another aspect provides a hand washing enclosure comprising:
an opening configured to receive an insertion of one or more hands of a user;
at least one input fixture configured to introduce one or more fluids to the hand washing enclosure;
at least one mobile washing tool; and
a conveyor configured to move the at least one mobile washing tool into proximity with the one or more hands of the user.

The hand washing enclosure may comprise: at least one visual indicator configured to indicate a stage or mode of the hand washing enclosure.

The at least one input fixture may comprise: at least one water input pipe configured to introduce water to the hand washing enclosure; and a soap input tube configured to introduce soap to the hand washing enclosure.

The at least one mobile washing tool may comprise a brush.

The at least one mobile washing tool may comprise a high pressure sprayer.

The hand washing enclosure may comprise: a mobile drying tool configured to direct air towards the one or more hands of the user.

The conveyer may be configured to move the mobile drying tool into proximity with the one or more hands of the user.

The hand washing enclosure may comprise: a drain configured to remove the one or more fluids from the hand washing enclosure.

The hand washing enclosure may comprise: a controller; and a timer that measures time elapsed from the insertion of one or more hands of a user.

The controller may open a valve for the at least one input fixture in response to the time elapsed from the insertion of one or more hands of a user.

The controller may actuate the conveyer in response to the time elapsed from the insertion of one or more hands of a user.

The hand washing enclosure may comprise: a user input device configured to receive a command from the user for operation of the hand washing enclosure, wherein the command from the user includes a mode selection, an activation for the hand washing enclosure, a temperature setting for the hand washing enclosure, or a duration for the hand washing enclosure.

The hand washing enclosure may comprise: an indicator responsive to operation of the hand washing enclosure.

The indicator may communicate a mode selection, an activation for the hand washing enclosure, a temperature setting for the hand washing enclosure, or a duration for the hand washing enclosure.

Another aspect provides a method for operation of a hand washing enclosure comprising: opening a valve for at least one input fixture configured to introduce one or more fluids to the hand washing enclosure; and actuating a conveyor to move at least one mobile washing tool into proximity with one or more hands of a user.

The method may comprise: detecting one or more hands of a user, wherein opening the valve or actuating the conveyor is triggered in response to detection of the one or more hands of the user.

The method may comprise: illuminating at least one visual indicator configured to indicate a stage or mode of the hand washing enclosure.

The at least one input fixture may comprise: at least one water input pipe configured to introduce water to the hand washing enclosure; and a soap input tube configured to introduce soap to the hand washing enclosure.

The method may comprise: receiving a command from the user for operation of the hand washing enclosure.

Opening the valve or actuating the conveyor may be triggered in response to the command from the user.

Another aspect provides an apparatus for hygienic air barriers, the apparatus comprising:
a fan configured to create an air flow; and
a duct configured to accelerate and direct the air flow as an air curtain to isolate a bathroom device from surroundings of the bathroom device.

The apparatus may comprise: a vacuum aligned with the air curtain and configured to exhaust the air current away from the surroundings of the bathroom device.

Another aspect provides a system for hygienic air barriers, the system comprising:
a fan configured to create an air flow; and
a distribution assembly configured to divide and direct the air flow into multiple paths;
a duct at each of the multiple paths configured to accelerate a divided air flow as an air curtain; and
a plurality of air curtain outputs each configured to isolate a respective bathroom device from surroundings of the bathroom device.

Another aspect provides a method for operating a system of hygienic air barriers, the method comprising:
detecting a location of one or more users with a presence sensor;
selecting one or more air curtains in response to the location of one or more users;
actuating a distribution device in response to the selected one or more air curtains; and
directing air flows for the one or more air curtains to isolate the one or more users.

## Claims

1. A method for monitoring hygiene, the method comprising:
receiving at least one hygiene criterion at a mobile device associated with a supervising user;
collecting hygiene performance data related to a hygiene activity a participating user via a sensor;
performing an analysis of the hygiene performance data according to at least one hygiene criterion; and
calculating a score for the participating user based on the analysis of the hygiene performance data.

2. The method of claim 1, further comprising:
providing the score to the mobile device associated with the supervising user.

3. The method of claim 1 or claim 2, further comprising:
providing the score to an interface associated with the sensor.

4. The method of claim 3, wherein the interface is associated with a mirror, optionally wherein the interface includes augmented reality comprising a reflection in the mirror and at least one graphic for the at least one hygiene criterion displayed in association with the reflection in the mirror.

5. The method of any one of claims 1 to 4, further comprising:
providing an incentive to the participating user in response to the score.

6. The method of claim 5, wherein the incentive includes a visual cue in proximity to the hygiene activity.

7. The method of claim 5 or claim 6, wherein the mobile device for the supervising user is a first mobile device, and wherein the incentive includes an access time for the participating user with a second mobile device.

8. The method of any one of claims 1 to 7, further comprising:
Providing a nonvisual cue to the participating user in response to the score, optionally wherein the nonvisual cue includes an audio message or a scent.

9. The method of any one of claims 1 to 8, wherein the at least one hygiene criterion includes a time value for the hygiene activity of the participating user, a sequence of events for the hygiene activity of the participating user and/or a schedule for the hygiene activity of the participating user.

10. The method of any one of claims 1 to 9, further comprising:
receiving at least one weight from the mobile device associated with the supervising user, wherein the score is calculated based on the at least one weight.

11. The method of any one of claims 1 to 10, further comprising:
determining an identifier for the participating user; and
storing the score with the identifier for the participating user.

12. A system for monitoring hygiene, the system comprising:
a first mobile device associated with a supervising user, the first mobile device configured to receive at least one hygiene criterion;
a second mobile device associated with a participating user, the second mobile device configured to collect hygiene performance data related to a hygiene activity of the participating user; and
a server configured to perform an analysis the hygiene performance data according to the at least one hygiene criterion and calculate a score for the participating user based on the analysis of the hygiene performance data.

13. An apparatus for monitoring hygiene, the apparatus comprising:
a communication interface configured to receive at least one hygiene criterion from a mobile device associated with a supervising user and receive hygiene performance data related to a hygiene activity a participating user via a sensor; and
a controller configured to perform an analysis the hygiene performance data according to at least one hygiene criterion and calculate a score for the participating user based on the analysis of the hygiene performance data.

14. The apparatus of claim 13, wherein the at least one hygiene criterion includes a time value for the hygiene activity of the participating user, a sequence of events for the hygiene activity of the participating user, and/or a schedule for the hygiene activity of the participating user.

15. The apparatus of claim 13 or claim 14, wherein the controller is configured to identify the participating user and select an incentive based on the score and the participating user, optionally wherein the apparatus is a server in communication with the mobile device associated with the supervising user.
